# EUROPEAN PATENT SPECIFICATION

(11) **EP 0 508 034 B1**
(45) Date of publication and mention of the grant of the patent: **28.02.1996**
(21) Application number: 91870206.9
(22) Date of filing: 13.12.1991
(51) Int. Cl.: C11D 3/37, C11D 17/06

(54) **Compact detergent composition containing polyvinylpyrrolidone**
Polyvinylpyrrolidon enthaltendes Kompaktwaschmittel
Composition détergente contenant des polyvinylpyrrolidones

(30) Priority: 12.04.1991 EP 91870062
(43) Date of publication of application: 14.10.1992
(73) Proprietor: THE PROCTER & GAMBLE COMPANY, Cincinnati, Ohio 45202 (US)
(72) Inventor: McCorquodale, Finlay, B-1200 Woluwe-St.-Lambert (BE); Williams, Timothy, Newcastle upon Tyne (GB); Busch, Alfred, B-2910 Londerzeel (BE)
(74) Representative: Canonici, Jean-Jacques

(56) References cited:
- EP-A- 0 011 501
- EP-A- 0 256 696
- WO-A-91/15566
- FR-A- 2 303 074
- GB-A- 2 137 221
- US-A- 3 000 830

## Description

### Technical Field

The present invention relates to compact detergent compositions which are specifically designed for the washing of coloured fabrics.

### Background

Laundry detergent compositions are well known in the art and it is known to use vinylpyrrolidone polymers in such compositions, as described in EP-A-262 897 and EP-A-256 696. GB-2-137-221 describes particulate built detergent compositions comprising polyvinylpyrrolidone and a soil releasing copolymer of polyethylene terephtulate and polyoxyethylene terephtulate. It is also known to use such polymers in detergent compositions specifically designed for the washing of colored fabrics, as in EP-A-265 257 which discloses compositions containing a vinyl-pyrrolidone polymer, a carboxylate polymer and a carboxy methyl cellulose, and EP-A-372 291 which describes detergent compositions comprising a specific surfactant mixture and a vinyl pyrrolidone polymer. FR-A-2303074 describes detergent compositions comprising a combination of complexing agents and protective colloids(eg. PVP).

In these compositions, the vinylpyrrolidone polymers are of a high molecular weight, typically from 15000 to 50000.

On the other hand, a new generation of detergent compositions is now being marketed, which can be best pictured as "compact detergents" although they have been given a variety of trade names such as "Ultra", "Supra", "Micro" ... The particularity of such detergent compositions is their relatively high density compared to conventional detergent compositions, and their ability to achieve the same efficiency than conventional detergent compositions by using a considerably lesser amount of "compact" detergent composition. This particularity is best reflected, in terms of composition, by a relatively low amount of inorganic filler salt. The efficiency of such "compact" detergent compositions is best achieved by eliminating the pre-wash cycle and by using dispersing and diffusing devices, which are put directly in the drum of the washing machine at the start of the main washing cycle.

The present invention is based on the discovery that in such a "compact" matrix vinyl pyrrolidone polymers of high molecular weight such as referred-to hereinabove, inhibit the removal of particulate soils from fabrics;

The present invention provides a solution to this newly discovered problem, by using polyvinyl pyrrolidones of a narrowly defined molecular weight in a compact detergent matrix, resulting in good dye-transfer inhibition benefits without impairment of particulate soil removal.

### Summary of the Invention

The present invention provides a granular detergent composition which is free of phosphate compounds comprising an anionic surfactant and a builder, said granular detergent composition containing no more than 15%, preferably 10% by weight of inorganic filler salt, and said granular detergent composition having a density of 550 to 950, preferably 650 to 850 g/litre of composition, characterized in that it contains polyvinylpyrrolidone of a molecular weight of from 8000 to 15000 at a level in the finished product so as to deliver from 5 to 500 mg/l, preferably 15 to 150 mg/l of said polyvinylpyrrolidone in the wash solution.

### Detailed Description of the Invention

The present detergent compositions are in granular form and are characterized by their density, which is higher than the density of conventional detergent compositions. The density of the compositions herein ranges from 550 to 950g/liter, preferably 650 to 850 g/liter of composition, measured at 20°C.

The "compact" form of the compositions herein is best reflected, in terms of composition, by the amount of inorganic filler salt; inorganic filler salts are conventional ingredients of detergent compositions in powder form; In conventional detergent compositions, the filler salts are present in substantial amounts, typically 17-35% by weight of the total composition.

In the present compositions, the filler salt is present in amounts not exceeding 15% of the total composition, preferably not exceeding 10%, most preferably not exceeding 5% by weight of the composition.

Inorganic filler salts, such as meant in the present compositions are selected from the alkali and alkaline-earth-metal salts of sulphates and chlorides.

A preferred filler salt is sodium sulphate.

### SURFACTANT

A wide range of surfactants can be used in the detergent compositions whereby anionic surfactants must be present. A typical listing of anionic, nonionic, ampholytic and zwitterionic classes, and species of these surfactants, is given in US Patent 3,664,961 issued to Norris on May 23, 1972.

Mixtures of anionic surfactants are particularly suitable herein, especially mixtures of sulphonate and sulphate surfactants in a weight ratio of from 5:1 to 1:2, preferably from 3:1 to 2:3, more preferably from 3:1 to 1:1. Preferred sulphonates include alkyl benzene sulphonates having from 9 to 15, especially 11 to 13 carbon atoms in the alkyl radical, and alpha-sulphonated methyl fatty acid esters in which the fatty acid is derived from a C₁₂-C₁₈ fatty source preferably from a C₁₆-C₁₈ fatty source. In each instance the cation is an alkali metal, preferably sodium. Preferred sulphate surfactants are alkyl sulphates having from 12 to 18 carbon atoms in the alkyl radical, optionally in admixture with ethoxy sulphates having from 10 to 20, preferably 10 to 16 carbon atoms in the alkyl radical and an average degree of ethoxylation of 1 to 6. Examples of preferred alkyl sulphates herein are tallow alkyl sulphate, coconut alkyl sulphate, and C₁₄₋₁₅ alkyl sulphates. The cation in each instance is again an alkali metal cation, preferably sodium.

One class of nonionic surfactants useful in the present invention are condensates of ethylene oxide with a hydrophobic moiety to provide a surfactant having an average hydrophilic-lipophilic balance (HLB) in the range from 8 to 17, preferably from 9.5 to 13.5, more preferably from 10 to 12.5. The hydrophobic (lipophilic) moiety may be aliphatic or aromatic in nature and the length of the polyoxyethylene group which is condensed with any particular hydrophobic group can be readily adjusted to yield a water-soluble compound having the desired degree of balance between hydrophilic and hydrophobic elements.

Especially preferred nonionic surfactants of this type are the C₉-C₁₅ primary alcohol ethoxylates containing 3-8 moles of ethylene oxide per mole of alcohol, particularly the C₁₄-C₁₅ primary alcohols containing 6-8 moles of ethylene oxide per mole of alcohol and the C₁₂-C₁₄ primary alcohols containing 3-5 moles of ethylene oxide per mole of alcohol.

Another class of nonionic surfactants comprises alkyl polyglucoside compounds of general formula

RO (CₙH₂ₙO)ₜZₓ

wherein Z is a moiety derived from glucose; R is a saturated hydrophobic alkyl group that contains from 12 to 18 carbon atoms; t is from 0 to 10 and n is 2 or 3; x is from 1.3 to 4, the compounds including less than 10% unreacted fatty alcohol and less than 50% short chain alkyl polyglucosides. Compounds of this type and their use in detergent are disclosed in EP-B 0 070 077, 0 075 996 and 0 094 118.

Also suitable as nonionic surfactants are poly hydroxy fatty acid amide surfactants of the formula 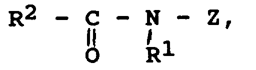 wherein R¹ is H, C₁₋₄ hydrocarbyl, 2-hydroxy ethyl, 2-hydroxy propyl or a mixture thereof, R₂ is C₅₋₃₁ hydrocarbyl, and Z is a polyhydroxyhydrocarbyl having a linear hydrocarbyl chain with at least 3 hydroxyls directly connected to the chain, or an alkoxylated derivative thereof. Preferably, R₁ is methyl, R₂ is a straight C₁₁₋₁₅ alkyl or alkenyl chain such as coconut alkyl or mixtures thereof, and Z is derived from a reducing sugar such as glucose, fructose, maltose, lactose, in a reductive amination reaction.

A further class of surfactants are the semi-polar surfactants such as amine oxides. Suitable amine oxides are selected from mono C₈-C₂₀, preferably C₁₀-C₁₄ N-alkyl or alkenyl amine oxides and propylene-1,3-diamine dioxides wherein the remaining N positions are substituted by methyl, hydroxyethyl or hydroxypropyl groups.

Another class of surfactants are amphoteric surfactants, such as polyamine-based species.

Cationic surfactants can also be used in the detergent compositions herein and suitable quaternary ammonium surfactants are selected from mono C₈-C₁₆, preferably C₁₀-C₁₄ N-alkyl or alkenyl ammonium surfactants wherein remaining N positions are substituted by methyl, hydroxyethyl or hydroxypropyl groups.

Mixtures of surfactant types are preferred, more especially anionic-nonionic and also anionic-nonionic-cationic mixtures. Particularly preferred mixtures are described in British Patent No. 2040987 and European Published Application No. 0 087 914. The detergent compositions can comprise from 1%-70% by weight of surfactant, but usually the surfactant is present in the compositions herein an amount of from 1% to 30%, more preferably from 10-25% by weight.

### BUILDER

Builder materials will typically be present at from 10% to 60% of the detergent compositions herein. The compositions herein are free or substantially free of phosphate-containing builders (substantially free being herein defined to constitute less than 1% of the total detergent builder system), and the builder system herein consists of water-soluble builders, water-insoluble builders, or mixtures thereof.

Water insoluble builders can be an inorganic ion exchange material,commonly an inorganic hydrated aluminosilicate material, more particularly a hydrated synthetic zeolite such as hydrated Zeolite A, X, B or HS.

Preferred aluminosilicate ion-exchange materials have the unit cell formula

M_{Z} [(A10₂)_{z} (SiO₂)_{y}] xH₂O

wherein M is a calcium-exchange cation, z and y are at least 6; the molar ratio of z to y is from 1.0 to 0.5 and x is at least 5, preferably from 7.5 to 276, more preferably from 10 to 264. The aluminosilicate materials are in hydrated form and are preferably crystalline containing from 10% to 28%, more preferably from 18% to 22% water.

The above aluminosilicate ion exchange materials are further charaterized by a particle size diameter of from 0.1 to 10 micrometers, preferably from 0.2 to 4 micrometers. The term "particle size diameter" herein represents the average particle size diameter of a given ion exchange material as determined by conventional analytical techniques such as, for example, microscopic determination utilizing a scanning electron microscope. The aluminosilicate ion exchange materials are further characterized by their calcium ion exchange capacity, which is at least 200 mg equivalent of CaCO₃ water hardness/g of aluminosilicate, calculated on an anhydrous basis, and which generally is in the range of from 300 mg eq./g to 352 mg eq./g. The aluminosilicate ion exchange materials herein are still further characterized by their calcium ion exchange rate which is described in detail in GB-1,429,143.

Aluminosilicate ion exchange materials useful in the practice of this invention are commercially available and can be naturally occurring materials, but are preferably synthetically derived. A method for producing aluminosilicate ion exchange materials is discussed in US Patent No. 3,985,669. Preferred synthetic crystalline aluminosilicate ion exchange materials useful herein are available under the designation Zeolite A, Zeolite B, Zeolite X, Zeolite HS and mixtures thereof. In an especially preferred embodiment, the crystalline aluminosilicate ion exchange material is Zeolite A and has the formula

Na₁₂[(A10₂)₁₂ (SiO₂)₁₂] xH₂O

wherein x is from 20 to 30, especially 27. Zeolite X of formula Na₈₆ [(A10₂)₈₆(SiO₂)₁₀₆] - 10.276 H₂O is also suitable, as well as Zeolite HS of formula Na₆ [(A10₂)₆(SiO₂)₆] 7.5 H₂O).

Another suitable water-insoluble, inorganic builder material is layered silicate, e.g. SKS-6 (Hoechst). SKS-6 is a crystalline layered silicate consisting of sodium silicate (Na₂Si₂O₅). The high Ca⁺⁺/Mg⁺⁺ binding capacity is mainly a cation exchange mechanism. In hot water, the material becomes more soluble.

The water-soluble builder can be a monomeric or oligomeric carboxylate chelating agent.

Suitable carboxylates containing one carboxy group include lactic acid, glycollic acid and ether derivatives thereof as disclosed in Belgian Patent Nos. 831,368, 821,369 and 821,370. Polycarboxylates containing two carboxy groups include the water-soluble salts of succinic acid, malonic acid, (ethylenedioxy) diacetic acid, maleic acid, diglycollic acid, tartaric acid, tartronic acid and fumaric acid, as well as the ether carboxylates described in German Offenlegenschrift 2,446,686, and 2,446,687 and U.S. Patent No. 3,935,257 and the sulfinyl carboxylates described in Belgian Patent No. 840,623. Polycarboxylates containing three carboxy groups include, in particular, water-soluble citrates, aconitrates and citraconates as well as succinate derivatives such as the carboxymethyloxysuccinates described in British Patent No. 1,379,241, lactoxysuccinates described in Netherlands Application 7205873, and the oxypolycarboxylate materials such as 2-oxa-1,1,3-propane tricarboxylates described in British Patent No. 1,387,447.

Polycarboxylates containing four carboxy groups include oxydisuccinates disclosed in British Patent No. 1,261,829, 1,1,2,2-ethane tetracarboxylates, 1,1,3,3-propane tetracarboxylates and 1,1,2,3-propane tetracarboxylates. Polycarboxylates containing sulfo substituents include the sulfosuccinate derivatives disclosed in British Patent Nos. 1,398,421 and 1,398,422 and in U.S. Patent No. 3,936,448, and the sulfonated pyrolysed citrates described in British Patent No. 1,082,179, while polycarboxylates containing phosphone substituents are disclosed in British Patent No. 1,439,000.

Alicyclic and heterocyclic polycarboxylates include cyclopentane-cis,cis,cis-tetracarboxylates, cyclopentadienide pentacarboxylates, 2,3,4,5-tetrahydrofuran - cis, cis, cis-tetracarboxylates, 2,5-tetrahydrofuran -cis - dicarboxylates, 2,2,5,5-tetrahydrofuran - tetracarboxylates, 1,2,3,4,5,6-hexane hexacarboxylates and and carboxymethyl derivatives of polyhydric alcohols such as sorbitol, mannitol and xylitol. Aromatic polycarboxylates include mellitic acid, pyromellitic acid and the phtalic acid derivatives disclosed in British Patent No. 1,425,343.

Of the above, the preferred polycarboxylates are hydroxycarboxylates containing up to three carboxy groups per molecule, more particularly citrates.

Preferred builder systems for use in the present compositions include a mixture of a water-insoluble aluminosilicate builder such as zeolite A, and a water-soluble carboxylate chelating agent such as citric acid.

Other builder materials that can form part of the builder system for the purposes of the invention include inorganic materials such as alkali metal carbonates, bicarbonates, silicates, and organic materials such as the organic phosphonates, amino polyalkylene phosphonates and amino polycarboxylates.

### The vinylpyrrolidone polymer

The composition herein also comprises a polyvinylpyrrolidone having a narrowly defined molecular weight of from 8000 to 15000. The selection of such a narrow molecular weight range provides unexpected benefits in terms of particulate soil removal. The level in the compositions according to the present invention of polyvinylpyrrolidone should be such that the amount of polyvinylpyrrolidone delivered in the wash solution is from 5 to 500 mg/l, preferably from 15 to 150 mg/l, most preferably 25 mg/l to 100 mg/l.

### OPTIONAL INGREDIENTS

The present compositions will typically include optional ingredients that normally form part of detergent compositions; Enzymes, antiredeposition and soil suspension agents, optical brighteners, bleaches, bleach activators, suds suppressors, anticacking agents, dyes and pigments are examples of such optional ingredients and can be added in varying amounts as desired.

Enzymes such as cellulase, proteases, lipases, or amylases are particularly desirable ingredients of the compositions herein.

Antiredeposition and soil suspension agents suitable herein include cellulose derivatives such as methylcellulose, carboxymethylcellulose and hydroxyethylcellulose, and homo- or co-polymeric polycarboxylic acids or their salts. Polymers of this type include the polyacrylates and maleic anhydride-acrylic acid copolymers previously mentioned as builders, as well as copolymers of maleic anhydride with ethylene, methylvinyl ether or methacrylic acid, the maleic anhydride constituting at least 20 mole percent of the copolymer. These materials are normally used at levels of from 0.5% to 10% by weight, more preferably from 0.75% to 8%, most preferably from 1% to 6% by weight of the composition.

Preferred optical brighteners are anionic in character, examples of which are disodium 4,4¹-bis-(2-diethanolamino-4-anilino -s- triazin-6-ylamino)stilbene-2:2¹ disulphonate, disodium 4, - 4¹-bis-(2-morpholino-4-anilino-s-triazin-6-ylaminostilbene-2:2¹ - disulphonate, disodium 4,4¹- bis-(2,4-dianilino-s-triazin-6-ylamino)stilbene-2:21 - disulphonate, monosodium 4¹,4¹¹ - bis-(2,4-dianilino-s-triazin-6 ylamino)stilbene-2-sulphonate, disodium 4,4¹ -bis-(2-anilino-4-(N-methyl-N-2-hydroxyethylamino)-s-triazin-6-ylamino)stilbene-2,2¹ -disulphonate, disodium 4,4¹ -bis-(4-phenyl-2,1,3-triazol-2-yl)-stilbene-2,2¹ disulphonate, disodium 4,4¹bis(2-anilino-4-(1-methyl-2-hydroxyethylamino)-s-triazin-6-ylamino)stilbene-2,2¹disulphonate and sodium 2(stilbyl-4¹¹-(naphtho-1¹,2¹:4,5)-1,2,3 - triazole-2¹¹-sulphonate.

Any particulate inorganic perhydrate bleach can be used, in an amount of from 3% to 40% by weight, more preferably from 8% to 25% by weight and most preferably from 12% to 20% by weight of the compositions. Preferred examples of such bleaches are sodium perborate monohydrate and tetrahydrate, percarbonate, and mixtures thereof.

Another preferred separately mixed ingredient is a peroxy carboxylic acid bleach percursor, commonly referred to as a bleach activator, which is preferably added in a prilled or agglomerated form. Examples of suitable compounds of this type are disclosed in British Patent Nos. 1586769 and 2143231 and a method for their formation into a prilled form is described in European Published Patent Application No. 0 062 523. Preferred examples of such compounds are tetracetyl ethylene diamine and sodium 3, 5, 5 trimethyl hexanoyloxybenzene sulphonate.

Bleach activators are normally employed at levels of from 0.5% to 10% by weight, more frequently from 1% to 8% and preferably from 2% to 6% by weight of the composition.

Another optional ingredient is a suds suppressor, exemplified by silicones, and silica-silicone mixtures. Silicones can be generally represented by alkylated polysiloxane materials while silica is normally used in finely divided forms exemplified by silica aerogels and xerogels and hydrophobic silicas of various types. These materials can be incorporated as particulates in which the suds suppressor is advantageously releasably incorporated in a water-soluble or water-dispersible, substantially non-surface-active detergent impermeable carrier. Alternatively the suds suppressor can be dissolved or dispersed in a liquid carrier and applied by spraying on to one or more of the other components.

As mentioned above, useful silicone suds controlling agents can comprise a mixture of an alkylated siloxane, of the type referred to hereinbefore, and solid silica. Such mixtures are prepared by affixing the silicone to the surface of the solid silica. A preferred silicone suds controlling agent is represented by a hydrophobic silanated (most preferably trimethyl-silanated) silica having a particle size in the range from 10 millimicrons to 20 millimicrons and a specific surface area above 50 m/g intimately admixed with dimethyl silicone fluid having a molecular weight in the range from about 500 to about 200,000 at a weight ratio of silicone to silanated silica of from about 1:1 to about 1:2.

A preferred silicone suds controlling agent is disclosed in Bartollota et al. U.S. Patent 3,933,672. Other particularly useful suds suppressors are the self-emulsifying silicone suds suppressors, described in German Patent Application DTOS 2,646,126 published April 28, 1977. An example of such a compound is DC-544, commercially availably from Dow Corning, which is a siloxane/glycol copolymer.

The suds suppressors described above are normally employed at levels of from 0.001% to 2% by weight of the composition, preferably from 0.01% to 1% by weight. The incorporation of the suds mofidiers is preferably made as separate particulates, and this permits the inclusion therein of other suds controlling materials such as C20-C24 fatty acids, microcrystalline waxes and high MW copolymers of ethylene oxide and propylene oxide which would otherwise adversely affect the dispersibility of the matrix. Techniques for forming such suds modifying particulates are disclosed in the previously mentioned Bartolotta et al U.S. Patent No. 3,933,672.

Other useful polymeric materials are the polyethylene glycols, particularly those of molecular weight 1000-10000, more particularly 2000 to 8000 and most preferably about 4000. These are used at levels of from 0.20% to 5% more preferably from 0.25% to 2.5% by weight. These polymers and the previously mentioned homo- or co-polymeric polycarboxylate salts are valuable for improving whiteness maintenance, fabric ash deposition, and cleaning performance on clay, proteinaceous and oxidizable soils in the presence of transition metal impurities.

Soil release agents useful in compositions of the present invention are conventionally copolymers or terpolymers of terephthalic acid with ethylene glycol and/or propylene glycol units in various arrangements. Examples of such polymers are disclosed in the commonly assigned US Patent Nos. 4116885 and 4711730 and European Published Patent Application No. 0 272 033. A particular preferred polymer in accordance with EP-A-0 272 033 has the formula

(CH₃(PEG)₄₃)_{0.75}(POH)_{0.25}[T-PO)_{2.8}(T-PEG)_{0.4}]T(PO-H)_{0.25}((PEG)₄₃CH₃)_{0.75}

where PEG is -(OC₂H₄)O-,PO is (OC₃H₆O) and T is (pcOC₆H₄CO).

Certain polymeric materials such as polyvinyl pyrrolidones typically of MW 5000-20000, preferably 10000-15000, also form useful agents in preventing the transfer of labile dyestuffs between fabrics during the washing process.

Fabric softening agents can also be incorporated into detergent compositions in accordance with the present invention. These agents may be inorganic or organic in type. Inorganic softening agents are exemplified by the smectite clays disclosed in GB-A-1,400,898. Organic fabric softening agents include the water-insoluble tertiary amines as disclosed in GB-A-1514276 and EP-B-0 011 340 and their combination with mono C12-C14 quaternary ammonium salts are disclosed in EP-B-0 026 527 and EP-B-0 026 528 and di-long-chain amides as disclosed in EP-B-0 242 919. Other useful organic ingredients of fabric softening systems include high molecular weight polyethylene oxide materials as disclosed in EP-A-0 299 575 and 0 313 146.

Levels of smectite clay are normally in the range from 5% to 20%, more preferably from 8% to 15% by weight with the material being added as a dry mixed component to the remainder of the formulation. Organic fabric softening agents such as the water-insoluble tertiary amines or di-long-chain amide materials are incorporated at levels of from 0.5% to 5% by weight, normally from 1% to 3% by weight whilst the high molecular weight polyethylene oxide materials and the water-soluble cationic materials are added at levels of from 0.1% to 2%, normally from 0.15% to 1.5% by weight. These materials are normally added to the spray dried portion of the composition, although in some instances it may be more convenient to add them as a dry mixed particulate, or spray them as a molten liquid on to other solid components of the composition.

### PROCESS OF WASHING

The compact detergent compositions herein have the ability to achieve the same efficiency than conventional detergent compositions, when a considerably lesser amount of composition herein, is used in the main wash cycle of a washing machine.

Accordingly, in an other embodiment of the invention, it is herewith provided for a process for washing fabrics in a washing machine wherein an amount of from 15 to 170 g of a detergent composition according to the present invention is used for the main wash cycle.

Typically, under European conditions, the recommended usage is from 80 to 140 g of detergent composition for the main wash cycle, without the need of a pre-wash.

The detergent compositions herein are preferably delivered directly to the drum and not indirectly via the outer casing of the machine. This can most easily be achieved by incorporation of the composition in a bag or container from which it can be released at the start of the wash cycle in response to agitation, a rise in temperature or immersion in the wash water in the drum. Such a container will be placed in the drum, together with the fabrics to be washed. Alternatively the washing machine itself may be adapted to permit direct addition of the composition to the drum e.g. by a dispensing arrangement in the access door.

Products comprising a detergent composition enclosed in a bag or container are usually designed in such a way that container integrity is maintained in the dry state to prevent egress of the contents when dry, but are adapted for release of the container contents on exposure to a washing environment, normally on immersion in an aqueous solution.

Usually the container will be flexible, such as a bag or pouch. The bag may be of fibrous construction coated with a water impermeable protective material so as to retain the contents, such as is disclosed in European published Patent Application No. 0 018 678. Alternatively it may be formed of a water insoluble synthetic polymeric material provided with an edge seal or closure designed to rupture in aqueous media as disclosed in European published Patent Application Nos. 0 011 500, 0 011 501, 0 011 502, and 0 011 968. A convenient form of water frangible closure comprises a water soluble adhesive disposed along and sealing one edge of a pouch formed of a water impermeable polymeric film such as polyethylene or polypropylene.

In a variant of the bag or container product form, laminated sheet products can be employed in which a central flexible layer is impregnated and/or coated with a composition and then one or more outer layers are applied to produce a fabric-like aesthetic effect. The layers may be sealed together so as to remain attached during use or may separate on contact with water to facilitate the release of the coated or impregnated material.

An alternative laminate form comprises one layer embossed or deformed to provide a series of pouch-like containers into each of which the detergent components are deposited in measured amounts, with a second layer overlying the first layer and sealted thereto in those areas between the pouch-like containers where the two layers are in contact. The components may be deposited in particulate, paste or molten form and the laminate layers should prevent egress of the contents of the pouch-like containers prior to their addition to water. The layers may separate or may remain attached together on contact with water, the only requirement being that the structure should permit rapid release of the contents of the pouch-like containers into solution. The number of pouch-like containers per unit area of substrate is a matter of choice but will normally vary between 500 and 25,000 per square metre.

Suitable materials which can be used for the flexible laminate layers in this aspect of the invention include, among others, sponges, paper and woven and non-woven fabrics.

However the preferred means of carrying out the washing process according to the present invention includes the use of a reusable dispensing device having walls that are permeable to liquid but impermeable to the solid composition.

Devices of this kind are disclosed in European Patent Application Publication Nos. 0 343 069 and 0 344 070. The latter Application discloses a device comprising a flexible sheet in the form of a bag extending from a support ring defining an orifice, the orifice being adapted to admit to the bag sufficient product for one washing cycle in a washing cycle. A portion of the washing medium flows through the orifice into the bag, dissolves the product, and the solution then passes outwardly through the orifice into the washing medium. The support ring is provided with a masking arrangement to prevent egress of wetted, undissolved, product, this arrangement typically comprising radially extending walls extending from a central boss in a spoked wheel configuration, or a similar structure in which the walls have a helical form.

### EXAMPLES

The following examples illustrate the invention and facilitate its understanding.

The abbreviations for the individual ingredients have the following meaning :
LAS: sodium salt of linear dodecyl benzene sulfonate
TAS: sodium salt of tallow alcohol sulfate
AS: sodium salt of alkyl ( C14 - C15 ) sulfate
AO: C12 - C14 alkyl dimethylamine oxide
FA45E7: fatty alcohol ( C14 - C15 ) ethoxylated with about 7 moles of ethylene oxide
CAT: C12 alkyl trimethyl ammonium chloride
Clay: smectite clay
Zeolite 4A: sodium salt of zeolite 4A with average particle size between 1 - 10 micrometer
SKS-6: crystalline layered silicate (Hoechst)
Copolymer AA/MA: copolymer of acrylic acid and maleic acid
CMC: carboxymethylcellulose
Phosphonate: sodium salt of ethylenediamine tetramethylene phosphonic acid
EDTA: sodium salt of ethylenediamine tetra acetate
PB1: NaBO2.H2O2
TAED: tetra acetyl ethylene diamine
P.A.: sulphonated zinc phthalocyanine
Silicate ( R = n ): SiO2 / Na20 = n
Amylase: Termamyl® 60T ( Novo-Nordisk )
Lipase: Lipolase® 100T ( Novo-Nordisk )
Protease: Savinase® 4T ( Novo-Nordisk )
SSS : Suds Suppressing System (silica/silicone mixture)

### Example I

The following compact detergent composition was prepared:

| Ingredients | % by weight |
|---|---|
| LAS | 9% |
| TAS | 3% |
| FA45E7 | 2.5% |
| Zeolite | 33% |
| Sodium Citrate | 21% |
| Sodium Carbonate | 3% |
| Sodium Silicate | 3% |
| Sodium Sulphate | 5% |
| PAA | 3.5% |
| Protease | 1.6% |
| Polyvinyl pyrrolidone | 1% |

### Testing procedure

a) A bundle of soiled fabrics containing fabrics which were stained with particulate soil was split into two parts and each part was washed in a compact detergent with the formulation which is detailed below. One half of each bundle was washed with the detergent composition of Example I, and one half was washed with the detergent composition of Example I without polyvinylpyrrolidone. Washing was done at 60°C and 90°C.

Eight such washes were done at each temperature. Particulate stain removal was evaluated in each case in terms of a panel score unit determined by two judges as follows :
1. I think there is a difference
2. There is definitely a difference
3. There is a big difference
4. There is a black and white difference

The average data, comparing the fabrics washed with polyvinlypyrrolidone vs. those washed without polyvinylpyrrolidone of different molecular weights is as follows :

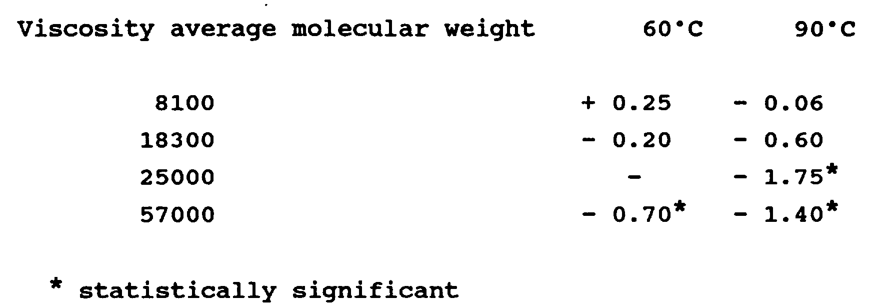

b) The dye transfer inhibition performance of the above compact detergent formulation was evaluated in the presence and absence of the above polyvinylpyrrolidone types by washing a clean, white, cotton and polyester load together with a red cotton garment, dyed with a sulphur/reactive dye combination, which bled dye into the wash. The resulting discolouration of cotton fabrics in the rest of the load was measured by Hunter Lab and the data are reported below as delta C values (=(a2 + b2)0.5). The experiment was repeated with a brown fabric dyed with a direct dye which bled into the wash.

| | red item | brown item |
|---|---|---|
| No PVP | 30.1 | 13.7 |
| 8100 | 25.5 | 4.6 |
| 18300 | -21.0 | - |
| 25000 | - | - |
| 57000 | 23.0 | 3.9 |

| | | |
|---|---|---|
| * statistically significant | | |

These data show that polyvinylpyrrolidone with molecular weight in the range 8-18000 gives dye transfer inhibition without a defficiency on particulate stain removal.

## Claims

1. A granular detergent composition which is free of phosphate compounds, further comprising an anionic surfactant and a builder, said granular detergent composition containing no more than 15% by weight of inorganic filler salt, and said granular detergent composition having a density of 550 to 950 g/litre of composition, characterized in that it contains polyvinylpyrrolidone of a molecular weight of from 8000 to 15000, at a level in the finished product so as to deliver from 5 to 500 mg/l of said polyvinylpyrrolidone in the wash solution.

2. A detergent composition according to claim 1 wherein the polyvinylpyrrolidone is present at a level so as to deliver from 15 mg/l to 150 mg/l of polyvinylpyrrolidone in the wash solution, preferably from 25 mg/l to 100 mg/l.

3. A detergent composition according to claims 1-2 wherein said inorganic filler salt is selected from alkali and alkaline-earth metal salts of sulphate and chloride.

4. A detergent composition according to claims 1-3 which does not contain more than 10% by wt of inorganic filler salt.

5. A detergent composition according to claims 1-4 which has a density of 650 to 850 g/liter.

6. A detergent composition according to claims 1-5 wherein said builder is selected from aluminosilicate ion exchangers, citrates, carbonates and mixtures thereof.

7. A detergent composition according to claims 1-6 which contains one or more enzymes, selected from proteases, cellulases, lipases, amylases and mixtures thereof.

8. A process for washing fabrics in a washing machine wherein an amount of from 15 to 170 g of a detergent composition according to claims 1-7 is used for the main wash cycle.

9. A process for washing fabrics according to claim 8 wherein said amount of detergent composition is put in a container able to release the composition at the start of the wash cycle, and said container is placed in the drum of the washing machine, together with the fabrics to be washed.

## Patentansprüche

1. Granulierte Waschmittelzusammensetzung, die frei an Phosphatverbindungen ist, umfassend weiterhin ein anionisches Tensid und einen Builder, wobei die granulierte Waschmittelzusammensetzung nicht mehr als 15 Gew-% eines anorganischen Füllstoffsalzes enthält und die granulierte Waschmittelzusammensetzung eine Dichte von 550 bis 950 g/Liter der Zusammensetzung aufweist, **dadurch gekennzeichnet**, daß sie Polyvinylpyrrolidon mit einem Molekulargewicht von 8000 bis 15000 in einem solchen Anteil im fertigen Produkt enthält, daß 5 bis 500 mg/l des Polyvinylpyrrolidons in die Waschlösung abgegeben werden.

2. Waschmittelzusammensetzung nach Anspruch 1, wobei das Polyvinylpyrrolidon in einem solchen Anteil vorliegt, daß 15 mg/l bis 150 mg/l Polyvinyl-pyrrolidon, vorzugsweise 25 mg/l bis 100 mg/l, in die Waschlösung abgegeben werden.

3. Waschmittelzusammensetzung nach den Ansprüchen 1 und 2, wobei das anorganische Füllstoffsalz aus Alkali- und Erdalkalimetallsalzen in Form von Sulfat und Chlorid gewählt ist.

4. Waschmittelzusammensetzung nach den Ansprüchen 1 bis 3, welche nicht mehr als 10 Gew.-% anorganisches Füllstoffsalz enthält.

5. Waschmittelzusammensetzung nach den Ansprüchen 1 bis 4, welche eine Dichte von 650 bis 850 g/l aufweist.

6. Waschmittelzusammensetzung nach den Ansprüchen 1 bis 5, wobei der Builder aus Aluminosilikat-Ionenaustauschern, Zitraten, Carbonaten und Mischungen hiervon gewählt ist.

7. Waschmittelzusammensetzung nach den Ansprüchen 1 bis 6, welche ein oder mehrere Enzyme, gewählt aus Proteasen. Cellulasen, Lipasen. Amylasen und Mischungen hiervon, enthält.

8. Verfahren zum Waschen von Textilien in einer Waschmaschine, wobei eine Menge von 15 bis 170 g einer Waschmittelzusammensetzung gemäß mindestens einem der Ansprüche 1 bis 7 für den Hauptwaschgang verwendet wird.

9. Verfahren zum Waschen von Textilien nach Anspruch 8, wobei die Menge der Waschmittelzusammensetzung in einen zur Freigabe der Zusammensetzung beim Start des Waschganges fähigen Behälter gegeben wird und der Behälter zusammen mit den zu waschenden Textilien in die Trommel der Waschmaschine eingesetzt wird.

## Revendications

1. Composition détergente granulaire ne contenant pas de phosphates, comprenant en outre un tensioactif anionique et un adjuvant, ladite composition détergente granulaire ne contenant pas plus de 15 % en poids d'un sel de charge minéral, et ladite composition détergente granulaire ayant une masse volumique de 550 à 950 g/litre de la composition, caractérisée en ce qu'elle contient une polyvinylpyrrolidone ayant une masse moléculaire de 8000 à 15 000, en une quantité, dans le produit fini, suffisante pour introduire dans la solution de lavage une quantité de 5 à 500 mg/l de ladite polyvinylpyrrolidone.

2. Composition détergente selon la revendication 1, dans laquelle la polyvinylpyrrolidone est présente en une quantité suffisante pour introduire dans la solution de lavage une quantité de polyvinylpyrrolidone de 15 à 150 mg/l et de préférence de 25 à 100 mg/l.

3. Composition détergente selon les revendications 1-2, dans laquelle ledit sel de charge minéral est choisi parmi les sulfates et chlorures de métaux alcalins et alcalino-terreux.

4. Composition détergente selon les revendications 1-3, qui ne contient pas plus de 10 % en poids d'un sel de charge minéral.

5. Composition détergente selon les revendications 1-4, qui a une masse volumique de 650 à 850 g/l.

6. Composition détergente selon les revendications 1-5, dans laquelle ledit adjuvant est choisi parmi les échangeurs d'ions de type aluminosilicate, les citrates, les carbonates et leurs mélanges.

7. Composition détergente selon les revendications 1-6, qui contient une ou plusieurs enzymes choisies parmi les protéases, les cellulases, les lipases, les amylases et leurs mélanges.

8. Procédé pour laver des textiles dans une machine à laver, dans lequel on utilise pour le cycle de lavage principal une quantité de 15 à 170 g d'une composition détergente selon les revendications 1-7.

9. Procédé pour laver des textiles selon la revendication 8, dans lequel ladite quantité de composition détergente est placée dans un récipient pouvant libérer la composition au début du cycle de lavage, ledit récipient étant placé dans le tambour de la machine à laver en même temps que les textiles à laver.
